# EUROPEAN PATENT SPECIFICATION

(11) **EP 3 970 625 B1**
(45) Date of publication and mention of the grant of the patent: **03.12.2025**
(21) Application number: 21197002.5
(22) Date of filing: 15.09.2021
(51) Int. Cl.: A61B 17/00

(54) **DEVICE FOR THE TREATMENT AND REGENERATION OF TISSUE FISTULAS**
VORRICHTUNG ZUR BEHANDLUNG UND REGENERIERUNG VON GEWEBEFISTELN
DISPOSITIF POUR LE TRAITEMENT ET LA REGENERATION DES FISTULES TISSULAIRES

(30) Priority: 18.09.2020 IT 202000022135
(43) Date of publication of application: 23.03.2022
(73) Proprietor: Tecnoss s.r.l., 10094 Giaveno (TO) (IT)
(72) Inventor: OLIVA, Giuseppe, 10094 Giaveno (TO) (IT)
(74) Representative: Buzzi, Notaro & Antonielli d'Oulx S.p.A.

(56) References cited:
- WO-A1-00/47143
- WO-A1-2021/188827
- WO-A1-94/13210
- WO-A2-2006/119256
- SU-A1- 1 204 193
- US-A1- 2005 019 262
- US-A1- 2008 245 374
- US-A1- 2011 282 354

## Description

### Field of the invention

The present invention relates to a medical device suitable for the treatment and resolution of fistulas in humans and animals.

### Technological background

By "fistula" is meant a pathological communication, of a tubular shape, between two structures or between two cavities of the organism or between these and the outside. Fistulas may involve practically all the organs of the entire body and form by diverse pathogenetic mechanisms.

The following types of fistulas may be distinguished:
- INTERNAL fistulas, where the pathological opening does not communicate with the outside (for example, a fistula between the gall bladder and the duodenum, where owing to inflammation of the gall bladder, with or without calculi, the wall thereof swells and transmits the inflammation to the nearby duodenum with necrosis of the contiguous tissues and genesis of the pathological communication between the two organs);
- EXTERNAL fistulas, where communication of one or two organs occurs towards the outside with a path that may concern the muscle planes, the adipose layers, and the skin; external fistulas constitute the majority of the fistulas and are identified according to the districts where they appear: for example, anal and perianal fistulas if the podex is concerned; sacro-coccigeal fistulas if the sacrum is concerned; dental or gengival fistulas if the maxillo-facial sector is concerned; cutaneous fistulas if the fistula is formed by appendages of the skin; and so forth.

### Causes, etiopathogenesis

Any inflammatory process aggravated by an infection can lead to the formation of a fistula; inflammation gives rise to the formation of an abscess, which once matured bursts, with exit of pus, the pus tending to drain off along a tract that traverses the tissues, which comes to constitute the fistula. If the fistula tract is single, the fistula is said to be "simple", but at times, by encountering tissue that offers resistance, other secondary tracts may open up, with or without outlet to the outside, giving rise to the so-called multiple fistula. Any organ may be subject to inflammation and hence to the formation of fistulas.

At times fistulas have a congenital nature as in the case of oesophago-bronchial fistulas, arteriovenous fistulas, or fistulas caused by cysts of the neck or thyroidal fistulas.

### Treatment of fistulas

In the simplest forms, medical treatment with anti-inflammatory agents and antibiotics is sufficient. In more serious forms medical treatment must be accompanied by surgical treatment, aimed at eliminating the primary cause of the fistula, the adhesions and/or the abscess, with a thorough cleansing of the area concerned and with closing upstream of the fistula tract, which in general is followed by cicatrisation or fibrosis.

Surgical treatment envisages an operation of removal of the fistula (fistulectomy), with various degrees of difficulty owing to its location and to the organs involved. Another type of surgical operation (fistulotomy) envisages, instead, simple opening-out or unroofing and draining of the fistula.

Surgical therapy is recommended only in certain cases given that the rate of success is around 60% for the initial operation (the fistula may re-present) and approaches 100% in the case where a person undergoes more than one operation.

At times the surgical operation must be preceded by the use of seton, a thin strand of linen or silk that is drawn through the fistula completely, such as to enable better draining and cleaning thereof to prepare it for the surgical operation.

At times, as in the case of cutaneous or facial fistulas, in order to prevent unseemly repair, fibrous repair, or even excessive apposition of keloid fibrous tissue, guided repair of the fistula tract is obtained by drawing along it medicated gauzes, or soft catheters, either synthetic ones or ones of animal origin, through which washing is performed with medications containing hypochlorite solutions (Amuchina) or iodides (Betadine), hydrogen peroxide, or medications with drugs, such as antibiotics, collagen-based cicatrizing agents, and hyaluronic acid.

The therapeutic purpose is to obtain a slow healing by secondary intention. Currently, in more aesthetically important areas, at the outlet of the fistula there are applied autologous haemoderivatives, i.e., ones deriving from the patient, or allologous haemoderivatives, i.e., ones deriving from a living donor, containing anti-inflammatory factors, growth factors, or even stem cells taken from adipose tissue so as to seek a complete healing, eliminating any inflammatory process that could be the cause of unseemly fibrosis or even recidivation of the fistula.

Widely used and with good results is fibrin glue, an allogeneic derivative obtained by selection from a pool of blood donors, which completely seals the walls of the fistula and induces proliferation of mesenchymal cells, which, by multiplying and differentiating in cells of the damaged tissue, lead to complete resolution of the fistula and to a *restitutio ad integrum.*

Other allologous preparations recently introduced and currently in the study stage consist in injectable solutions of mesenchymal stem cells derived from the adipose tissue of living donors (eASC), which are thus injected into the fistula tract that has been cleaned out millions of stem cells (Alofisel).

Surgical treatment of tissue fistulas hence consists in a curettage with thorough cleansing of the entire fistula tract. In order to favour healing and closing of the fistula tract and prevent recidivation thereof, medicated gauzes or collagen plugs, that have conical or conical-cylindrical shapes, or plugs made from animal tissues (collagen) or synthetic tissues (re-absorbable polymers) are used.

Plugs for surgical treatment of tissue fistulas are generally constituted by biomaterials of animal or synthetic origin. These are tiny cylinders or micro-cones formed by rolling of de-antigenated porcine submucosa, similar, in shape and structure, to a cigar (Surgisis AFP, manufactured by COOK MEDICAL INC.), or else made of polymeric material, such as polyglycolic acid and trimethylene-carbonate (GORE B10-A Plug). Plugs that have shapes substantially similar to the aforesaid commercial devices are illustrated, for example, in SU1204193, FR2840796, US2008/245374, US2011/282354, WO2014/205269, WO2006/119256 and WO00/47143. Even though these devices are excellent space retainers, frequently in longer fistulas that traverse tonic or strong muscle tissue, they undergo squeezing and fragmentation of their fibres, with consequent expulsion thereof through the external orifice of the fistula.

Recently, with patent EP 2916741B1 a plug has been provided, having an average length of 9-12 cm, indicated for the treatment of anal fistulas, which would seem to guarantee its own primary stability, and a good filling of the fistula tract, an excellent contact with the vascularized walls of the fistula, with adequate flow of blood to facilitate regeneration of demaged tissue. A particular aspect of this plug is its frustoconical shape, resembling an obelisk, with very pronounced sharp edges and notches that prevent movement thereof, both rotary movement and longitudinal movement, in the fistula tract. This device does not, however, constitute an optimal solution for the treatment of fistulas in so far as the pronounced edges of the structure of the device may lead, by compression, to an ischaemia and consequent necrosis of the tissues adjacent to the fistula, with consequent impossibility of healing of the fistula itself.

Plugs for surgical treatment of tissue fistulas constituted by a single element made of biomaterial of synthetic or natural origin, the single element being a body having a length, a width, a thickness and a longitudinal axis, the body having a proximal end portion and a distal end portion, wherein the body has a solid portion, a rounded distal proximal end portion, a rectangular cross section and rounded edges, the body being provided with a plurality of longitudinal cuts at the proximal distal end portion to obtain a lamellar structure are disclosed i.a. in WO 2021/188827.

WO 2006/119256 discloses a medical device for the treatment of fistulas having the features set forth in the preamble of claim 1.

### Summary of the invention

The foregoing having been said, there is hence felt the need to provide improved solutions that will enable provision of devices for the treatment of fistulas that will be able to overcome the disadvantages of the prior art.

According to one or more embodiments, this object can be achieved thanks to what is specified in the annexed claims, which form an integral part of the present description.

The present invention regards a medical device suitable for the treatment of fistulas, constituted by a single element made of biomaterial of synthetic or natural origin, the single element being a body having a length, a width, a thickness and a longitudinal axis, the body having a proximal end portion and a distal end portion, wherein the body has a rectangular cross section and rounded edges (A), has a solid portion for two thirds of its length, a rounded distal end portion, and in the remaining part of the body is provided with a plurality of longitudinal cuts (B) at the proximal end portion to obtain a lamellar structure, wherein the length is comprised between 1 cm and 12 cm, the width is comprised between 0.5 cm and 3 cm, and the thickness is comprised between 1 mm and 5 mm.

The longitudinal cuts B increase the surface of contact of the device with the fistula, enabling a greater adaptability of the device to the shape and size of the fistula itself. The entire distal third of the device according to the invention thus structured is in fact able to open up, with its lamellae, like an umbrella. The good adhesion of the medical device to the surface of the fistula enables proper healing of the latter. The longitudinal cuts B perform the following essential and innovative functions as compared to the plugs described above: a) they considerably increase the wettable and contact surface of the device given the same total volume of the plug, upgrading the function of carrier thereof; b) they perform a braking and blocking function in the case where by gravity the device were to be urged out of the cleansed fistula tract in so far as each lamella freely and autonomously adapts to the walls of the tract, interlacing with the others to form a terminal plug that is non-compressive but adheres to the walls.

### Brief description of the drawings

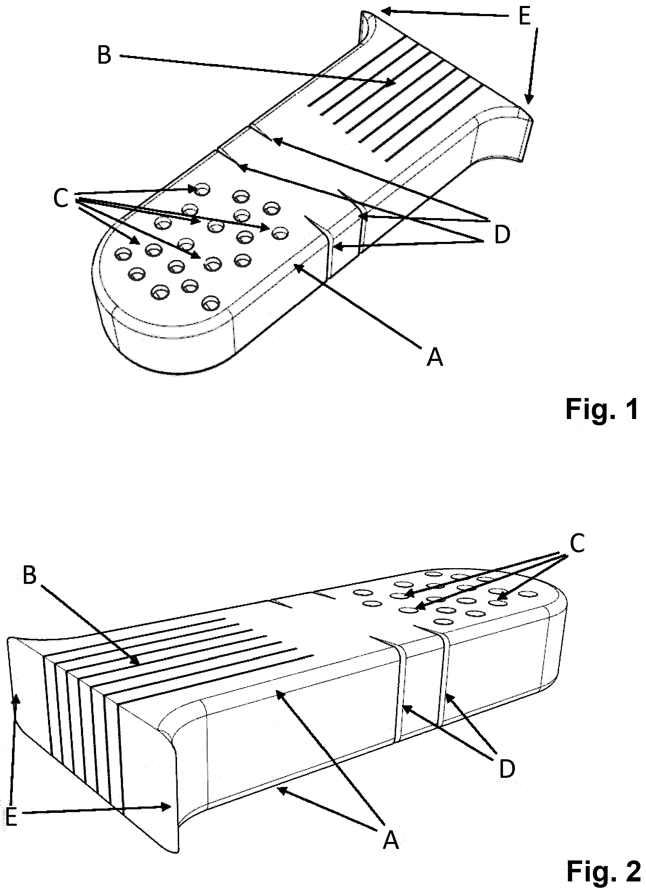

The invention will now be described in detail, purely by way of illustrative and non-limiting example and with reference to the attached drawings, wherein Figures 1 and 2 represent, in perspective view, a preferred embodiment of the medical device.

### Detailed description of the invention

In the ensuing description, numerous specific details are presented to enable a complete understanding of the embodiments. The embodiments may be implemented without one or more of the specific details, or with other methods, components, materials, etc. In other cases, well-known structures, materials, or operations are not illustrated or described in detail so that certain aspects of the embodiments will not be obscured.

Throughout the present specification, reference to "an embodiment" or "one embodiment" is used to mean that a particular configuration, structure, or characteristic described in connection with the embodiment is included in at least one embodiment. Hence, the use of phrases such as "in an embodiment" or "in a certain embodiment" that may be present in various points of the present specification does not necessarily refer to one and the same embodiment. Moreover, the details, configurations, structures, and characteristics may be combined in any suitable way in one or more embodiments.

The headings used herein are provided merely for convenience and do not express the purpose or meaning of the embodiments.

The present invention regards a medical device, also referred to as plug, for the treatment of fistulas.

In one embodiment, the medical device, which is designed for treating fistulas in humans and animals, is constituted by a single element made of biomaterial of synthetic or natural origin, the single element being a body having a length, a width, a thickness and a longitudinal axis, the body having a proximal end portion and a distal end portion, characterized in that the body has a rectangular cross section and rounded edges A and in that the body is provided with a plurality of longitudinal cuts B in the proximal end portion to obtain a lamellar structure.

In one embodiment, the medical device comprises (in the respective proximal end portion) an end stop plug E suitable for guaranteeing fixing of the device to the entry orifice of the fistula, even without suturing the device itself to the wall of the fistula. The end stop plug E enables, that is, the stability of the device necessary for integration and progressive re-absorption and remodelling of the device.

In one embodiment, the medical device comprises on its respective lateral surface wells C. These wells C (which are preferably present in the distal end portion) preferably have a diameter of 1 mm and different depths comprised between 1 mm and 3 mm. The wells C increase the surface of the device and enable, at the discretion of the surgeon, delivery of retarded-release drugs and/or platelet-rich growth factors.

In one embodiment, the medical device comprises lateral notches D substantially perpendicular to the longitudinal axis of the device. These lateral notches D (which are preferably present in the central portion of the device) enable an increase in the wettable surface of the device. The lateral notches D may moreover function as a guide for the surgeon in cutting the device in order to reduce the length thereof according to the need.

According to the invention, the medical device has a rectangular section and a dimension of length comprised between 1 cm and 12 cm, a dimension of width comprised between 0.5 cm and 3 cm, and a dimension of thickness comprised between 1 mm and 5 mm.

The medical device forming the subject of the present application is shaped like a long-necked funnel, of rectangular cross section, compact, with rounded edges and its lateral surface pitted with wells.

This device may moreover function as carrier or sponge for drugs, and/or haemoderivatives, and/or stem cells, chosen at the discretion of the medical practitioner, because it is able to receive them and transmit them via its pitted surface and its frayed terminal part.

The device is a slow-reabsorption device in order to ensure formation a soft scar, i.e., a scar that is not fibrous or, even worse, of a hyperproductive keloid type, with perfect *restitutio ad integrum* in more aesthetically important and exposed areas.

Thanks to its shape, the above device opens up new prospects in the treatment of fistulas in general, irrespective of the characteristic and location of the fistulas treated and of the biomaterial of which it is made. The device may in fact be constituted by material of animal origin, i.e., obtained from porcine, bovine, or equine skin rendered biocompatible, or else of synthetic origin, i.e., obtained with slow-reabsorption polymers (e.g., polyglycolic-polyurethane acid).

Thanks to its rounded edges, the device overcomes the technical problem linked to the genesis of areas of tissue suffering caused by the medical devices known to the art, which, by presenting sharp edges, exert circumscribed and anomalous pressure on the cleansed tissue, with formation of necrotic areas, secondary to pressure sores caused by the sharp edges.

With reference to Figures 1 and 2, the medical device forming the subject of the present invention has a rectangular cross section and is characterized by:
(A) rounded edges (i.e., without sharp edges);
(B) longitudinal cuts at the base of the plug;
(C) wells present on the surfaces of the plug;
(D) lateral notches; and
(E) end stop plugs.

The plug in question hence presents primary characteristics that, in addition to differentiating it from the existing forms, affords new possibilities for improvement of the treatment of fistulas.

The device is a plug made of any biocompatible biomaterial, whether of synthetic or animal origin, in the form of a long-necked funnel, of rectangular section (Figures 1 and 2), with rounded edges, i.e., without sharp edges A in order to prevent anomalous areas of pressure on the cleansed tissue and the secondary formation of possible pressure sores.

The body of the plug is constituted by a solid portion for two thirds of its length, with a rounded distal end (apical) portion, whereas in the remaining part, towards the base, it has longitudinal cuts B, which, by generating a lamellar structure, bestow a soft consistency on the entire base/terminal part of the plug.

This part cut into soft lamellae B moreover solves the technical problem of the impossibility of soaking the plug with selected solutions (drugs, antibiotics, growth factors, platelet derivatives, etc.) after its grafting into the fistula tract; this portion in fact, following upon how it is treated in the production stage, presents disorganized and receptive in regard to solutions or derivatives.

The solid portion of the plug has wells C, purposely formed so that they have a diameter of 1 mm but different depths (from 1 mm to 3 mm) that increase the surface thereof and enable, at the discretion of the surgeon, resolution of the problem of managing to deliver, via the plug, retarded-release drugs and/or platelet-rich growth factors (denoted as PRP, PRF, PRGF), thus stimulating regeneration of tissue and activating maturation of blasts.

The lateral portion of the edges presents notches D, which have the function of increasing the wettability of the device according to the invention, in addition to providing a guide for the surgeon to perform possible operations of cutting and shortening of the plug.

The distal portion of the device is shaped like a plug E designed to guarantee fixing of the plug to the entry orifice, even without any need for it to be sutured to the wall of the fistula, so as to guarantee the stability necessary for integration and progressive re-absorption and remodelling of the plug.

The plug forming the subject of the present description preferably has the following dimensions:
- length: from 1 cm to 12 cm;
- average width: between 0.5 cm and 3 cm; and
- thickness: from 1 mm to 5 mm.

The variant embodiment of synthetic origin is obtained with the use of dies pre-formed so as to be the negative of the device shown in Figure 1 and Figure 2, into which the polymer used is injected under pressure.

The natural variant embodiment is obtained from animal skin that has been de-antigenated or rendered biocompatible. Its fabrication initially envisages cutting of the original tissues, with dies made of stainless steel 316, in order to obtain a first wedge-shaped semi-finished product, comprising plug shape (letter E in Figures 1 and 2) at the base of the device.

The semi-finished product is then exposed to an action of striking of the proximal end portion (base of the plug), aimed at obtaining microscopic fragmentation of the collagen and elastin fibres of this part. The semi-finished product is then exposed to a thermal treatment with refrigeration at -20°C for at least 1 hour. Using purposely designed steel slot milling cutters mounted on turbine handpieces, the edges of the frozen semi-finished product are then rounded (letter A in the figures) in order to eliminate the sharp edges of the semi-finished product. Next, provided on the surface of the semi-finished product, using cylindrical cutters with a diameter of 1 mm, are the wells of variable depth, but in no case are they through wells (letter C in the figures). With a purposely provided die carrying parallel end blades, using an expressly designed hydraulic precision press longitudinal cuts B are made right through the thickness, in the distal third of the device according to the invention. Once again using a hydraulic precision press, the lateral notches (letter D in the figures) are provided.

Once the device is completed, it undergoes quality control of a dimensional and mechanical nature.

Sterilization of the device according to the invention is obtained using gamma or beta radiation, or by exposure to ethylene oxide.

## Claims

1. A medical device suitable for the treatment of fistulas, constituted by a single element made of biomaterial of synthetic or natural origin, the single element being a body having a length, a width, a thickness and a longitudinal axis, the body having a proximal end portion and a distal end portion, wherein the body has a rectangular cross section and rounded edges (A), has a solid portion for two thirds of its length, a rounded distal end portion, and in the remaining part of the body it is provided with a plurality of longitudinal cuts (B) at the proximal end portion to obtain a lamellar structure, wherein the length is comprised between 1 cm and 12 cm, the width is comprised between 0.5 cm and 3 cm, and the thickness is comprised between 1 mm and 5 mm.

2. The medical device according to Claim 1, comprising an end stop plug (E) defined in the proximal end portion.

3. The medical device according to Claim 1 or Claim 2, wherein the body has a first lateral surface provided with wells (C).

4. The medical device according to any one of the preceding claims, wherein the body has two second lateral surfaces perpendicular to the first lateral surface, the two second lateral surfaces being provided with lateral notches (D) extending over the whole thickness of the body, the notches (D) being substantially perpendicular to the longitudinal axis.

## Patentansprüche

1. Medizinische Vorrichtung zur Behandlung von Fisteln, bestehend aus einem einzigen Element aus einem Biomaterial synthetischen oder natürlichen Ursprungs, wobei das einzige Element ein Körper mit einer Länge, einer Breite, einer Dicke und einer Längsachse ist, wobei der Körper einen proximalen Endabschnitt und einen distalen Endabschnitt aufweist, wobei der Körper einen rechteckigen Querschnitt und abgerundete Kanten (A) aufweist, zu zwei Dritteln seiner Länge einen massiven Abschnitt aufweist, einen abgerundeten distalen Endabschnitt aufweist und im verbleibenden Teil des Körpers am proximalen Endabschnitt mit einer Vielzahl von Längsschnitten (B) versehen ist, um eine lamellenförmige Struktur zu erhalten, wobei die Länge zwischen 1 cm und 12 cm liegt, die Breite zwischen 0,5 cm und 3 cm liegt und die Dicke zwischen 1 mm und 5 mm liegt.

2. Medizinische Vorrichtung nach Anspruch 1, mit einem Endanschlagstopfen (E), der im proximalen Endabschnitt definiert ist.

3. Medizinische Vorrichtung nach Anspruch 1 oder Anspruch 2, wobei der Körper eine erste Seitenfläche aufweist, die mit Vertiefungen (C) versehen ist.

4. Medizinische Vorrichtung nach einem der vorstehenden Ansprüche, wobei der Körper zwei zweite Seitenflächen senkrecht zur ersten Seitenfläche aufweist, wobei die beiden zweiten Seitenflächen mit seitlichen Kerben (D) versehen sind, die sich über die gesamte Dicke des Körpers erstrecken, wobei die Kerben (D) im Wesentlichen senkrecht zur Längsachse sind.

## Revendications

1. Dispositif médical adapté au traitement de fistules, constitué d'un élément unique fait d'un biomatériau d'origine synthétique ou naturelle, l'élément unique étant un corps ayant une longueur, une largeur, une épaisseur et un axe longitudinal, le corps ayant une portion d'extrémité proximale et une portion d'extrémité distale, dans lequel le corps a une section transversale rectangulaire et des bords arrondis (A), a une portion solide pour deux tiers de sa longueur, une portion d'extrémité distale arrondie, et, dans la partie restante du corps, il est doté d'une pluralité de découpes longitudinales (B) au niveau de la portion d'extrémité proximale pour que soit obtenue une structure lamellaire, dans lequel la longueur est comprise entre 1 cm et 12 cm, la largeur est comprise entre 0,5 cm et 3 cm, et l'épaisseur est comprise entre 1 mm et 5 mm.

2. Dispositif médical selon la revendication 1, comprenant un bouchon d'arrêt d'extrémité (E) défini dans la portion d'extrémité proximale.

3. Dispositif médical selon la revendication 1 ou la revendication 2, dans lequel le corps a une première surface latérale dotée de puits (C).

4. Dispositif médical selon l'une quelconque des revendications précédentes, dans lequel le corps a deux deuxièmes surfaces latérales perpendiculaires à la première surface latérale, les deux deuxièmes surfaces latérales étant dotées d'encoches latérales (D) s'étendant sur toute l'épaisseur du corps, les encoches (D) étant pratiquement perpendiculaires à l'axe longitudinal.
